# EUROPÄISCHE PATENTANMELDUNG

(11) **EP 1 950 269 A1**
(43) Veröffentlichungstag der Anmeldung: **30.07.2008**
(21) Anmeldenummer: 08000688.5
(22) Anmeldetag: 15.01.2008
(51) Int. Cl.: C10G 11/00, C10G 47/00, C07C 4/20

(54) **Verfahren zur Dampf-Dealkylierung**

(30) Priorität: 26.01.2007 DE 102007004077
(71) Anmelder: Linde Aktiengesellschaft, 80807 München (DE)
(72) Erfinder: Fritz, Helmut, 81373 München (DE); Göke, Volker, 82515 Wolfrathausen (DE); Ponceau, Marianne, 81371 München (DE); Schödel, Nicole, 81477 München (DE); Wenning, Ulrike, 82049 Pullach (DE)

(57) **Zusammenfassung**

Die Erfindung beschreibt ein Verfahren zur Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer mit Katalysatormaterial gefüllten und von außen beheizten Vorrichtung in einer Anlage zur Erzeugung von Kohlenwasserstoffen aus kohlenwasserstoffhaltigem Einsatz, wobei die Einsatzstoffe die Vorrichtung durchströmen und als Reaktionsprodukte aromatische Kohlenwasserstoffe und entsprechende Alkane neben gasförmigen Reaktionsprodukten vorwiegend bestehend aus Wasserstoff, Kohlenmonoxid, Kohlendioxid und Methan entstehen. In den Eingangsbereich der Vorrichtung zur Dampf-Dealkylierung wird zusätzlich zu den Einsatzstoffen der Dampf-Dealkylierung ein wasserstoffhaltiges Gas geführt, wodurch sich die Lebensdauer des Katalysatormaterials verlängert.

## Beschreibung

Die Erfindung betrifft ein Verfahren zur Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer mit Katalysatormaterial gefüllten und von außen beheizten Vorrichtung in einer Anlage zur Erzeugung von Kohlenwasserstoffen aus kohlenwasserstoffhaltigem Einsatz, wobei die Einsatzstoffe die Vorrichtung durchströmen und als Reaktionsprodukte aromatische Kohlenwasserstoffe und entsprechende Alkane neben gasförmigen Reaktionsprodukten vorwiegend bestehend aus Wasserstoff, Kohlenmonoxid, Kohlendioxid und Methan entstehen

In einer Anlage zur Erzeugung von Kohlenwasserstoffen aus kohlenwasserstoffhaltigem Einsatz entsteht ein Gemisch aus Kohlenwasserstoffen mit einer unterschiedlichen Anzahl von Kohlenstoffatomen und unterschiedlichen molekularen Konfigurationen. Dieses Gemisch wird in der Anlage zur Erzeugung von Kohlenwasserstoffen aus kohlenwasserstoffhaltigem Einsatz in die einzelnen Kohlenwasserstoffprodukte aufgetrennt.

Abhängig von der jeweiligen Trennsequenz und der Art der Anlage zur Erzeugung von Kohlenwasserstoffen aus kohlenwasserstoffhaltigem Einsatz entstehen eine Kohlenwasserstofffraktion mit mindestens sechs Kohlenstoffatomen (C₆₊-Fraktion) und/oder eine Kohlenwasserstofffraktion mit mindestens sieben Kohlenstoffatomen (C₇₊-Fraktion). Diese Fraktionen enthalten als wirtschaftlich verwertbares Produkt Aromaten, welche als Ausgangsstoff für die Synthese zahlreicher Kunststoffe und zur Erhöhung der Klopffestigkeit von Benzin Verwendung finden.

Um an die wirtschaftlich verwertbaren Produkte der C₆₊-Fraktion, vor allem Benzol, zu gelangen und die Ausbeute möglichst maximal zu gestalten, wird nach dem Stand der Technik folgendes Verfahren angewendet. Die C₆₊-Fraktion wird in eine Fraktion überwiegend bestehend aus Kohlenwasserstoffen mit sechs Kohlenstoffatomen (C₆-Fraktion) und eine C₇₊-Fraktion getrennt. Aus der C₆-Fraktion kann direkt das wirtschaftlich verwertbare Produkt Benzol abgetrennt werden. Aus der C₇₊-Fraktion werden mittels einer Flüssig-Flüssig Extraktion die linearen Kohlenwasserstoffe abgetrennt und als so genanntes Raffinat weiterverarbeitet. Die von den linearen Kohlenwasserstoffen befreite C₇₊-Fraktion enthält nunmehr hauptsächlich Aromaten mit sieben bis acht Kohlenstoffatomen und wird in eine Fraktion überwiegend bestehend aus Kohlenwasserstoffen mit sieben Kohlenstoffatomen (hauptsächlich Toluol) und in eine Fraktion überwiegend bestehend aus Kohlenwasserstoffen mit acht Kohlenstoffatomen (hauptsächlich Xylol) getrennt. Die Fraktion überwiegend bestehend aus Kohlenwasserstoffen mit acht Kohlenstoffatomen wird als Einsatzstoff in ein Verfahren zur para-Xylol Gewinnung geführt. Die Fraktion überwiegend bestehend aus Kohlenwasserstoffen mit sieben Kohlenstoffatomen wird als Einsatz in ein Verfahren zur Hydro-Dealkylierung geführt wird.

Ein derartiges Verfahren zur Hydro-Dealkylierung ist zum Beispiel in WO2005071045 beschrieben. Die Kohlenwasserstoffe werden in Anwesenheit eines Katalysators bei einer Temperatur von 400°C bis 650°C und einem Druck zwischen 20 bar und 40 bar mit Wasserstoff kontaktiert, wobei der Wasserstoff in einem molaren Überschuss vom drei- bis sechsfachen der Kohlenwasserstoffe vorliegt. Unter diesen Bedingungen werden die Alkylgruppen von den jeweiligen alkylierten Aromaten (wie zum Beispiel Toluol) abgespalten, so dass sich Benzol und die jeweiligen Alkane (zum Beispiel Methan) bilden.

Der Verbrauch von Wasserstoff bei der Hydro-Dealkylierung der Kohlenwasserstoffe wirkt sich negativ auf die Wirtschaftlichkeit dieses Verfahrens nach dem Stand der Technik zur Benzolgewinnung aus.

In einem alternativen Verfahren werden die C₆₊-Fraktion und/oder die C₇₊-Fraktion einer Dampf-Dealkylierung unterzogen (siehe zum Beispiel eingereichte Patentschriften DE102006058528.3 oder DE102006058529.1). Bei einer Dampf-Dealkylierung der C₆₊-Fraktion und/oder C₇₊-Fraktion entstehen hauptsächlich die beiden verwertbaren Produktstoffe Benzol und Wasserstoff neben Reaktionsprodukten wie Kohlenmonoxid und Kohlendioxid. Als Einsatzstoff der Dampf-Dealkylierung wird somit lediglich kostengünstiger Wasserdampf bei gleichzeitiger Produktion des gewünschten Wertproduktes Benzol und des wertvollen Nebenproduktes Wasserstoff benötigt.

In diesem Verfahren nach dem Stand der Technik reagieren die Kohlenwasserstoffe aus der jeweiligen Kohlenwasserstofffraktion mit Wasserdampf in der Gasphase an einem festen Katalysator. Dabei werden die Kohlenwasserstoffe und der Wasserdampf in einer Vorrichtung, zum Beispiel Rohre, an dem festen Katalysator vorbeigeführt, wobei sich der Katalysator im Inneren der Vorrichtung, zum Beispiel im Rohrinneren, befindet. Die für die Dealkylierung nötige Reaktionswärme wird der Vorrichtung von außen zugeführt, zum Beispiel durch Verbrennung der gasförmigen Reaktionsnebenprodukte Kohlenmonoxid und Methan mit Luft. Nach dem Stand der Technik entspricht die Temperatur der Einsatzstoffe der Temperatur der Reaktionsprodukte und liegt im Bereich von 400°C bis 600°C. Das Katalysatormaterial im Inneren der Vorrichtung besteht im Wesentlichen aus einem porösen Trägermaterial wie γ-Al₂O₃, MgAl Spinell und/oder Cr₂O₃, und einer an der Oberfläche des Trägermaterials befindlichen Aktivkomponente, wie Rh mit 0.1-1.0 Gew% Beladung und/oder Pd mit 0.2-2.0 Gew% Beladung. Aus den gasförmigen Reaktionsprodukten der Dampf-Dealkylierung wird über eine Druckwechseladsorption mit vorheriger Verdichtung der wirtschaftlich verwertbare Wasserstoff von den Reaktionsnebenprodukten wie Kohlenmonoxid, Kohlendioxid und Methan getrennt.

Bei der Reaktion der Einsatzstoffe am aktiven katalytischen Zentrum des Katalysatormaterials werden zu einem bestimmten Anteil neben den eigentlichen Reaktionsprodukten auch höhermolekulare Kohlenstoffverbindungen gebildet, die teilweise als Ablagerungen auf dem Katalysatormaterial verbleiben. Die sich ablagernden höhermolekularen Kohlenstoffverbindungen blockieren die Möglichkeit zur Adsorption der Edukte auf der Katalysatoroberfläche und führen so zu einer mit der Zeit zunehmenden Deaktivierung (Verkokung). Die Verkokungsgeschwindigkeit ist dabei in erster Näherung linear zum Partialdruck der Kohlenwasserstoffe in der Gasphase.

Der Prozess der Verkokung ist reversibel. Nach dem Stand der Technik wird ein so verkoktes Katalysatormaterial in einem Abstand von mehreren Wochen von den Ablagerungen durch Abbrennen unter Zufuhr von Sauerstoff oder sauerstoffhaltigen Gasen befreit und somit regeneriert. Alternativ zum Abbrennen kann das Katalysatormaterial mit einem Einsatzstoff aus Wasserdampf und Wasserstoff begast werden, wodurch die Kohlenstoffablagerungen ebenfalls vom Katalysatormaterial entfernt werden. Diese Verfahrensweise ist durch die verringerte Gefahr der Ausbildung von lokal sehr heißen Stellen schonender und wirkt sich damit positiv auf die Lebensdauer des Katalysators aus. Bei beiden Verfahren zur Regeneration des Katalysatormaterials muss die Zufuhr der Kohlenwasserstofffraktion und somit die Produktion des gewünschten Wertproduktes Benzol gestoppt werden. Diese Produktionsstopps zur Regeneration des Katalysatormaterials wirken sich negativ auf die Betriebskosten des Verfahrens aus.

Der vorliegenden Erfindung liegt daher die Aufgabe zugrunde, ein Verfahren der eingangs erwähnten Art derart auszugestalten, das die Deaktivierung des Katalysatormaterials weitestgehend vermieden beziehungsweise verlangsamt wird, wodurch die Betriebskosten des Verfahrens sinken und der Produkterlös zugleich gesteigert wird (weniger Regenerations-, mehr Produktionszeit).

Die vorliegende Aufgabe wird dadurch gelöst, dass in den Eingangsbereich der Vorrichtung ein Gas geführt wird, wobei das zugeführte Gas reich an Wasserstoff ist und mit Eingangsbereich der Teil der Vorrichtung bezeichnet wird, der als erstes von den Einsatzstoffen durchströmt wird.

Es hat sich gezeigt, dass die Verkokung des aktiven Zentrums des Katalysatormaterials auch vom herrschenden Partialdruck des Wasserstoffs in der Gasphase über dem Katalysatormaterial abhängig ist. Die Verkokung des aktiven Zentrums ist bei erhöhtem Wasserstoffpartialdruck deutlich geringer. Bei der Dampf-Dealkylierung der Kohlenwasserstofffraktion entsteht neben den dealkylierten Aromaten und den entsprechenden Alkanen auch Wasserstoff. Im Eingangsbereich der Vorrichtung startet die Reaktion, so dass hier primär nur die Einsatzstoffe vorhanden sind und sich das Reaktionsprodukt Wasserstoff erst bildet beziehungsweise sich nur in geringem Maß gebildet hat. Daher tritt im Eingangsbereich der Vorrichtung, wo der niedrigste Wasserstoffpartialdruck herrscht, verstärkt Verkokung auf. Kann die Verkokung im Eingangsbereich vermieden werden, kann somit auch die Deaktivierung des Katalysators hier verlangsamt werden und die Intervalle bis zu einem nötigen Produktionsstopp zur Regeneration des Katalysatormaterials vergrößern sich.

Durch die Anwendung des erfindungsgemäßen Verfahrens wird der Wasserstoffpartialdruck am aktiven Zentrum des Katalysatormaterials deutlich gegenüber dem Stand der Technik erhöht und damit die Verkokung des Eingangsbereiches deutlich minimiert.

In einer Ausgestaltung der Erfindung wird in den Eingangsbereich der Vorrichtung gasförmiger Wasserstoff geführt. Vorteilhafterweise wurde der zugeführte Wasserstoff in der Vorrichtung zur Dampf-Dealkylierung erzeugt. Für das erfindungsgemäße Verfahren kann Wasserstoff aus einer beliebigen Quelle der Anlage oder der aus der Dampf-Dealkylierung erzeugte Wasserstoff verwendet werden. Die gasförmigen Reaktionsprodukte der Dampf-Dealkylierung bestehen hauptsächlich aus Wasserstoff.

In einer bevorzugten Ausgestaltung der Erfindung werden die gasförmigen Reaktionsprodukte in einem mehrstufigen Verdichtungsprozess verdichtet und anschließend mittels eines Druckwechseladsorptionsverfahrens in Wasserstoff und einen gasförmigen Rest überwiegend bestehend aus Kohlenmonoxid, Kohlendioxid und Methan getrennt. Der mittels Druckwechselabsorptionsverfahren abgetrennte Wasserstoff wird vorteilhafterweise zumindest teilweise in den Eingangsbereich der Vorrichtung geführt.

In einer anderen Ausgestaltung der Erfindung wird zumindest ein Teil der gasförmigen Reaktionsprodukte vorwiegend bestehend aus Wasserstoff, Kohlenmonoxid, Kohlendioxid und Methan aus einer der Verdichterstufen ausgeführt und als wasserstoffreiches Gas in den Eingangsbereich der Vorrichtung geführt. Aus dem überwiegenden Teil der gasförmigen Reaktionsprodukte kann nach der abgeschlossenen Verdichtung mittels eines Druckwechseladsorptionsverfahrens reiner Wasserstoff abgetrennt und in anderen Teilen der Anlage oder außerhalb der Anlage verwertet werden. In dieser Ausgestaltung der Erfindung enthält das wasserstoffreiche Gas auch Kohlenmonoxid. Im Eingangsbereich ist ohne die Zufuhr von Kohlenmonoxid noch kein Kohlenmonoxid vorhanden beziehungsweise hat sich Kohlenmonoxid nur in geringem Maß gebildet. Die Adsorption von Kohlenmonoxid auf der Oberfläche des Katalysatormaterials ist hier ebenfalls möglich und wirkt sich positiv auf die Unterdrückung von unerwünschten Nebenreaktionen, wie zum Beispiel die vollständige Vergasung der Aromaten, aus. Durch diese Ausgestaltung der Erfindung wird eine Steigerung der Selektivität der Dampf-Dealkylierung erreicht.

In einer weiteren Ausgestaltung wird zumindest ein Teil der gasförmigen Reaktionsprodukte vorwiegend bestehend aus Wasserstoff, Kohlenmonoxid, Kohlendioxid und Methan aus einer der Verdichterstufen ausgeführt und über ein Verfahren zur Kohlendioxidabreicherung als wasserstoffreiches Gas in den Eingangsbereich der Vorrichtung geführt. In dieser Ausgestaltung der Erfindung wird das Gesamtvolumen des in den Eingangsbereich der Vorrichtung zuzuführenden Gases reduziert. Das aus den gasförmigen Reaktionsprodukten durch das Verfahren zur Kohlendioxidabreicherung abgetrennte kohlendioxidreiche Gas kann als Einsatzstoff in die nächste Verdichterstufe zurückgeführt werden.

Zweckmäßigerweise wird das wasserstoffreiche Gas im Gemisch mit den Einsatzstoffen der Dampf-Dealkylierung in den Eingangsbereich der Vorrichtung geführt, wobei das molare Verhältnis von Wasserstoff zu Wasser zwischen 0.005 und 0.5, bevorzugt zwischen 0.01 und 0.3, besonders bevorzugt zwischen 0.05 und 0.1, liegt.

Mit der vorliegenden Erfindung gelingt es insbesondere die Lebenszeit des Katalysatormaterials und den zeitlichen Abstand zwischen Produktionsstopps zur Katalysatorregeneration zu verlängern. Dadurch werden die Betriebskosten des Verfahrens deutlich verringert.

## Patentansprüche

1. Verfahren zur Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer mit Katalysatormaterial gefüllten und von außen beheizten Vorrichtung in einer Anlage zur Erzeugung von Kohlenwasserstoffen aus kohlenwasserstoffhaltigem Einsatz, wobei die Einsatzstoffe die Vorrichtung durchströmen und als Reaktionsprodukte aromatische Kohlenwasserstoffe und entsprechende Alkane neben gasförmigen Reaktionsprodukten vorwiegend bestehend aus Wasserstoff, Kohlenmonoxid, Kohlendioxid und Methan entstehen, **dadurch gekennzeichnet, dass** in den Eingangsbereich der Vorrichtung ein Gas geführt wird, wobei das zugeführte Gas wasserstoffhaltig ist und mit Eingangsbereich der Teil der Vorrichtung bezeichnet wird, der als erstes von den Einsatzstoffen durchströmt wird.

2. Verfahren nach Anspruch 1, **dadurch gekennzeichnet, dass** in den Eingangsbereich der Vorrichtung gasförmiger Wasserstoff geführt wird.

3. Verfahren nach Anspruch 1 oder 2, **dadurch gekennzeichnet, dass** der zugeführte Wasserstoff in der Vorrichtung zur Dampf-Dealkylierung erzeugt wurde.

4. Verfahren nach einem der Ansprüche 1 bis 3, **dadurch gekennzeichnet, dass** die gasförmigen Reaktionsprodukte in einem mehrstufigen Verdichtungsprozess verdichtet werden.

5. Verfahren nach Anspruch 4, **dadurch gekennzeichnet, dass** die verdichteten gasförmigen Reaktionsprodukte mittels eines Druckwechseladsorptionsverfahrens in Wasserstoff und einen gasförmigen Rest überwiegend bestehend aus Kohlenmonoxid, Kohlendioxid und Methan getrennt werden.

6. Verfahren nach Anspruch 5, **dadurch gekennzeichnet, dass** der mittels Druckwechselabsorptionsverfahren abgetrennte Wasserstoff zumindest teilweise in den Eingangsbereich der Vorrichtung geführt wird.

7. Verfahren nach Anspruch 4, **dadurch gekennzeichnet, dass** zumindest ein Teil der gasförmigen Reaktionsprodukte vorwiegend bestehend aus Wasserstoff, Kohlenmonoxid, Kohlendioxid und Methan aus einer der Verdichterstufen ausgeführt und als wasserstoffreiches Gas in den Eingangsbereich der Vorrichtung geführt wird.

8. Verfahren nach Anspruch 4, **dadurch gekennzeichnet, dass** zumindest ein Teil der gasförmigen Reaktionsprodukte vorwiegend bestehend aus Wasserstoff, Kohlenmonoxid, Kohlendioxid und Methan aus einer der Verdichterstufen ausgeführt und über ein Verfahren zur Kohlendioxidabreicherung als wasserstoffreiches Gas in den Eingangsbereich der Vorrichtung geführt wird.

9. Verfahren nach einem der Ansprüche 1 bis 3, **dadurch gekennzeichnet, dass** das wasserstoffreiche Gas im Gemisch mit den Einsatzstoffen der Dampf-Dealkylierung in den Eingangsbereich der Vorrichtung geführt wird, wobei das molare Verhältnis von Wasserstoff zu Wasser zwischen 0.005 und 0.5, bevorzugt zwischen 0.01 und 0.3, besonders bevorzugt zwischen 0.05 und 0.1, liegt.
